## Europäisches Patentamt
## European Patent Office
## Office européen des brevets

(19)

(11) Veröffentlichungsnummer: **0 090 308**
**B2**

(12) # NEUE EUROPÄISCHE PATENTSCHRIFT

(45) Veröffentlichungstag der neuen Patentschrift:
08.06.88

(21) Anmeldenummer: 83102767.7

(22) Anmeldetag: 21.03.83

(51) Int. Cl.⁴: **C 07 C 69/54,** C 07 C 67/54,
**C 07 C 29/82**

(54) Verfahren zur Abtrennung von Methanol aus Wasser enthaltenden Mischungen von Methylmethacrylat und Methanol.

(30) Priorität: 31.03.82 DE 3211901

(43) Veröffentlichungstag der Anmeldung:
05.10.83 Patentblatt 83/40

(45) Bekanntmachung des Hinweises auf die Patenterteilung:
07.08.85 Patentblatt 85/32

(45) Bekanntmachung des Hinweises auf die Entscheidung
über den Einspruch:
08.06.88 Patentblatt 88/23

(84) Benannte Vertragsstaaten:
BE DE FR GB IT NL

(56) Entgegenhaltungen:
EP-A-0 044 409
US-A-2 406 561

Lee H. Horsley (ed.): Azeotropic Data-III,
Washington (American Chemical Society), 1973,
S.76 ( Advances in Chemistry Series 116)

(73) Patentinhaber: BASF Aktiengesellschaft, Carl-Bosch- Strasse 38, D-6700 Ludwigshafen (DE)

(72) Erfinder: Duembgen, Gerd, Dr., Sudetenstrasse 4,
D-6701 Dannstadt- Schauernheim (DE)
Erfinder: Fouquet, Gerd, Dr., Maxburgstrasse 2,
D-6730 Neustadt (DE)
Erfinder: Krabetz, Richard, Dr., Unterer Waldweg
8, D-6719 Kirchheim (DE)
Erfinder: Merger, Franz, Dr., Max- Slevogt- Strasse
25, D-6710 Frankenthal (DE)
Erfinder: Nees, Friedbert, Dr., Fichtestrasse 4,
D-7513 Stutensee (DE)

EP 0 090 308 B2

## Beschreibung

Bei der Veresterung von Methacrylsäure mit Methanol setzt man in der Regel einen Überschuß an Methanol ein, so daß man nach der Veresterungsreaktion das überschüssige Methanol wieder entfernen muß. Destilliert man derartige methanolhaltige Mischungen, so erhält man zunächst ein azeotropes Gemisch aus 89 Gew.-% Methanol und 11 Gew.-% Methylmethacrylat. Da hierbei Methylmethacrylat verlorengeht, ist diese Verfahrensweise unwirtschaftlich.

Auch bei der Herstellung von Methylmethacrylat durch katalytische Oxidation von Methacrolein in Gegenwart von Methanol, bei der gleichzeitig eine Veresterung der entstehenden Methacrylsäure stattfindet, stellt sich das Problem der Abtrennung von Methanol aus Wasser enthaltenden Mischungen, die - abgesehen von Nebenprodukten der Oxidation - als wesentliche Bestandteile Methanol, Methylmethacrylat und Wasser enthalten.

Aufgabe der Erfindung ist es, ein Verfahren zur Abtrennung von Methanol aus Mischungen, die gegebenenfalls Wasser enthalten und hauptsächlich aus Methylmethacrylat und Methanol bestehen, zur Verfügung zu stellen, bei dem praktisch keine Verluste an Methylmethacrylat auftreten.

Die Aufgabe wird erfindungsgemäß gelöst mit einem Verfahren zur Abtrennung von Methanol aus Wasser enthaltenden Mischungen von Methylmethacrylat und Methanol durch azeotrope Destillation des Methanols aus der Mischung, wenn man zu der Mischung als Azeotropbildner n-Octan, Toluol oder Dimethylcarbonat zusetzt.

Obwohl die Differenz der Siedepunkte der azeotropen Gemische aus Methylmethacrylat und Methanol (64,2° C) einerseits und den Azeotropbildnern mit Methanol andererseits (z. B. beträgt der Siedepunkt des azeotropen Gemisches aus Toluol und Methanol 63,8° C) sehr gering ist, gelingt es überraschenderweise, Methanol in Form des Azeotrops aus der Mischung zu entfernen und Methylmethacrylat sowie gegebenenfalls Wasser im Rückstand anzureichern. Angaben über Verbindungen, die mit Methanol binäre azeotrope Gemische bilden, sind in der Literatur vorhanden, vgl. L.H. Horsley, Azeotropic Data III, Advances in Chemistry Series 116, American Chemical Society, Washington D.C., 1973.

Das erfindungsgemäße Verfahren eignet sich zum Abtrennen von Methanol aus Mischungen von Methylmethacrylat, Methanol und gegebenenfalls Wasser. Diese Mischungen können beispielsweise 10 bis 95 Gew.-% Methanol enthalten. Das Vefahren hat vor allem Bedeutung für die Abtrennung von Methanol aus Wasser enthaltenden Mischungen, die bei der katalytischen Oxidation und gleichzeitigen Veresterung von Methacrolein in Gegenwart von Methanol anfallen. Solche Mischungen enthalten 1 bis 3 Gew.-% Wasser, 80 bis 95 Gew.-% Methanol und 5 bis 15 Gew.-% Methylmethacrylat.

Als Azeotropbildner werden der Mischung, aus der Methanol abgetrennt werden soll, Verbindungen zugesetzt, die in Gegenwart von Methylmethacrylat und gegebenenfalls Wasser mit Methanol ein Azeotrop bilden. Die Menge an Azeotropbildner, die bei dem erfindungsgemäßen Vefahren eingesetzt wird, ist vom Methanolgehalt des Gemisches, aus dem das Methanol abgetrennt werden soll, abhängig. Vorzugsweise ist die Menge an Azeotropbildner so groß, daß sie gerade dazu ausreicht, um das gesamte Methanol aus dem Gemisch abzudestillieren. Man kann jedoch auch ohne verfahrenstechnische oder wesentliche wirtschatftiche Nachteile in Kauf nehmen zu müssen, einen bis zu 10 gew.-%-igen Überschuß an Azeotropbildner, bezogen auf Methanol im Gemisch, verwenden.

Geeignete Azeotropbildner, die in Gegenwart von Methylmethacrylat und Wasser mit Methanol ein binäres Azeotrop ergeben, sind n-Octan, Toluol und Dimethylcarbonat. Der Siedepunkt der azeotropen Gemische aus den in Betracht kommenden Azeotropbildnern mit Methanol liegt um 0,2 bis 15 und vorzugsweise 0,4 bis 1,5 ° C unterhalb des Siedepunkts des Azeotrops aus Methylmethacrylat und Methanol. Der Anteil der genannten Azeotropbildner in der azeotropen Mischung liegt unter 31 Gew.-%.

Die Entfernung des Methanols aus methanolischen Lösungen von Methylmethacrylat unter Zusatz von Azeotropbildnern efolgt bei Normaldruck oder vorzugsweise unter vermindertem Druck, z. B. bei 100 bis 1 000 mbar, um eine zu diesem Zeitpunkt unerwünschte Polymerisation des Methylmethacrylats zu verhindern bzw. weitestgehend zu unterdrücken. Es ist allgemein üblich, die methanolischen Lösungen von Methylmethacrylat bzw. auch reines Methylmethacrylat vor dem Destillieren mit den dafür gebräuchlichen Stabilisatoren in den üblichen Mengen zu versetzen, um damit die Polymerisation des Methylmethacrylats zu inhibieren. Das methanolhaltige Azeotrop, das zunächst aus der Mischung abdestilliert und als Fraktion mit dem niedrigsten Siedepunkt am Kopf der Kolonne abgenommen wird, kann in den Fällen, in denen das Azeotrop im flüssigen Zustand zwei Phasen bildet, getrennt werden. Man erhält dann eine im wesentlichen aus Methanol bestehende Phase und eine im wesentlichen aus dem Azeotropbildner zusammengesetzte Phase, die in die Kolonne zurückgeführt werden kann. Falls die Destillationsanlage mit einer Anlage zur Herstellung von Methylmethacrylat durch katalytische Oxidation von Methacrolein in Gegenwart von Methanol gekoppelt ist, kann die am Kopf der Kolonne der Destillationsanlage abgenommene methanolreiche Fraktion zur Herstellung des Methylmethacrylats in die Produktionsanlage zurückgeführt werden, in der die Oxidation und Veresterung vorgenommen wird.

Es gibt jedoch auch Fälle, in denen sich das methanolhaltige Azeotrop, das am Kopf der Kolonne abgezogen wird, nicht in zwei Phasen trennt. In diesem Fällen kann man beispielsweise Methanol und den verwendeten Azeotropbildner mit Wasser behandeln, um das Methanol auszuwaschen und durch Destillation aus dem Waschwasser zurückzugewinnen. Das zurückgewonnene Methanol kann bei der Veresterung der Methacrylsäure wieder eingesetzt werden.

Sobald das Methanol und der zugesetzte Azeotropbildner bzw. auch eine Mischung von geeigneten Azeotropbildnern aus der Wasser enthaltenden Mischung von Methylmethacrylat und Methanol abdestilliert sind - Methylmethacrylat und Azeotropbildner sind mit Hilfe einer fraktionierten Destillation leicht trennbar - wird das im Rückstand befindliche Wasser, das sich als selbständige untere Phase vom Methylmethacrylat scheidet, abgetrennt. Danach kann das Methylmethacrylat einer Destillation unterworfen werden, um es in einer für die Polymerisation ausreichenden Reinheit zu gewinnen.

Die in den folgenden Beispielen beschriebenen Destillationen wurden in einer Füllkörperkolonne durchgeführt, die 1 m lang war und einen Durchmesser von 3,6 cm hatte. Sie war mit steglosen Dixon-Ringen (vgl. M. Huber und A. Sperandio, Chemie Ing. Techn., 36, 221-227, 1964) gefüllt, deren Durchmesser 3 mm betrug. In sämtlichen Beispielen wurde ein Rücklaufverhältnis von 10 eingestellt. Der Sumpf der Kolonne enthielt als Stabilisator 0,01 Gew.-% 4-Methoxyphenol, bezogen auf Methylmethacrylat. Außerdem leitete man während der Destillation stündlich 1 l Luft durch den Sumpf und die Kolonne. Die Zusammensetzung der Destillate wurde gaschromatographisch bestimmt. In allen Beispielen erhielt man einen Rückstand aus Methylmethacrylat und Wasser. Das Wasser konnte im Scheidetrichter vom Methylmethacrylat abgetrennt werden, das dann mit Hilfe einer Destillation in reiner Form hergestellt wurde. Die Angaben in % beziehen sich jeweils auf das Gewicht der Stoffe.

## Beispiel 1

Ein Gemisch aus 840 g Methanol, 160 g n-Octan, 85 g Methylmethacrylat und 15 g Wasser wurde so destilliert, daß am Kopf der Kolonne ein Azeotrop aus n-Octan und Methanol bei 63-63,5°C abgenommen werden konnte. Das abdestillierte Azeotrop trennte sich in der Vorlage in zwei Phasen, von denen die obere Phase aus 2 % Methanol und 98 % Octan zusammengesetzt war und in die Kolonne zurückgeführt wurde, während die untere Phase im wesentlichen aus Methanol bestand und abgetrennt wurde. Methylmethacrylat und Wasser bildeten den Destillationsrückstand.

## Bespiel 2

Ein Gemisch aus 710 g Methanol, 290 g Toluol, 71 g Methylmethacrylat und 13 g Wasser wurde derart fraktionierend destilliert, daß am Kopf der Kolonne bei einer Temperatur von 63,5-64°C ein azeotropes Gemisch aus Methanol und Toluol abgenommen werden konnte. Der Destillationsrückstand bestand aus 2 Phasen, wovon die untere, hauptsächlich aus Wasser bestehende, Phase abgetrennt wurde, während die obere Phase, die aus Toluol und Methylmethacrylat bestand, einer fraktionierten Destillation unterworfen wurde. Man erhielt bei einer Temperatur von 100°C reines Methylmethacrylat.

## Patentansprüche

1. Verfahren zur Abtrennung von Methanol aus Wasser enthaltenden Mischungen von Methylmethacrylat und Methanol durch azeotrope Destillation des Methanol aus der Mischung, dadurch gekennzeichnet, daß man zu der Mischung als Azetropbildner n-Octan, Toloul oder Dimethylcarbonat zusetzt.

2. Verfahren nach Anspruch 1, dadurch gekennzeichnet, daß Methanol aus Wasser enthaltenden Mischungen abgetrennt wird, die bei der katalytischen Oxidation und gleichzeitigen Veresterung von Methacrolein in Gegenwart von Methanol anfallen.

## Claims

1. A method of separating off methanol from a watercontaining mixture of methyl methacrylate and methanol by azeotropic distillation, wherein n-octane, toluene or dimethyl carbonate is added to the mixture as azeotrope former.

2. A method as claimed in claim 1, wherein methanol is separated off from a watercontaining mixture obtained in the catalytic oxidation of methacrolein in the presence of methanol, where esterification of the oxidation product also takes place.

## Revendications

1. Procédé pour l'extraction du méthanol de mélanges aqueux de méthanol et de méthacrylate de méthyle par une distillation azéotrope de l'alcool méthylique, caractérisé en ce que l'on ajoute au mélange, à titre de composé formateur d'azéotrope, du n-octane, du toluène ou du carbonate de diméthyle.

2. Procédé suivant la revendication 1, caractérisé en ce qu'il est utilisé pour l'extraction du méthanol de mélanges aqueux obtenus lors de l'oxydation catalysée de la méthacroléine avec estérification concomitante en présence d'alcool méthylique.